Europäisches Patentamt

European Patent Office

Office européen des brevets

(11) Publication number: **0 409 438 A1**

# EUROPEAN PATENT APPLICATION

(21) Application number: **90307204.9**

(22) Date of filing: **02.07.90**

(51) Int. Cl.5: **C12N 1/20, A01N 63/00,**
//(C12N1/20,C12R1:07)

The applicant has filed a statement in accordance with Rule 28 (4) EPC (issue of a sample only to an expert). Accession number(s) of the deposit(s): NRRL B-18514 and 18515.

(30) Priority: **21.07.89 US 384221**

(43) Date of publication of application:
**23.01.91 Bulletin 91/04**

(84) Designated Contracting States:
**AT BE CH DE DK ES FR GB GR IT LI LU NL SE**

(71) Applicant: **Mycogen Corporation**
**5451 Oberlin Drive**
**San Diego, CA 92121(US)**

(72) Inventor: **Olson, Theresa C.**
**12798 Rancho Penasquitos Blvd. No. 136**
**San Diego, California 92129(US)**
Inventor: **Payne, Jewel**
**7984 Hemphill Drive**
**San Diego, California 92126(US)**
Inventor: **Alby, Theodore**
**North 2400 Wilbur Road, Apt. 126**
**Spokane, Washington 99206(US)**
Inventor: **Talbot, Henry W.**
**4111 Sturgeon Court**
**San Diego, California 92130(US)**

(74) Representative: **Perry, Robert Edward et al**
**GILL JENNINGS & EVERY 53-64 Chancery**
**Laneane**
**London WC2A 1HN(GB)**

(54) **Novel Bacillus thuringiensis isolates.**

(57) The invention concerns novel Bacillus thuringiensis ( B.t. ) isolates which have activity against all mosquitoes tested. Parent B.t. isolates are treated to obtain lysis⁻ spo⁻ cry⁺ mutants which retain the biological activity of the parent B.t. isolates.

EP 0 409 438 A1

## NOVEL BACILLUS THURINGIENSIS ISOLATES

### Background of the Invention

Bacillus thuringiensis ( B.t. ) produces an insect toxin designated as δ-endotoxin. It is synthesized by the B.t. sporulating cell. The toxin, upon being ingested in its crystalline form by susceptible insect larvae, is transformed into biologically active moieties by the insect gut juice proteases. The primary target is insect cells of the gut epithelium, which are rapidly destroyed.

The reported activity spectrum of B.t. covers insect species within the orders Lepidoptera and Coleoptera, many of which are major pests in agriculture and forestry. The activity spectrum also includes the insect order Diptera, which includes mosquitoes and black flies. See Couch, T.L. (1980) "Mosquito Pathogenicity of Bacillus thuringiensis var. israelensis ," Developments in Industrial Microbiology 22:61-76; Beegle, C.C. (1978) "Use of Entomogenous Bacteria in Agroecosystems," Developments in Industrial Microbiology 20:97-104.

### Brief Summary of the Invention

The subject invention concerns novel Bacillus thuringiensis ( B.t. ) isolates which have activity against dipteran pests. For example, the novel B.t. isolates, known herein as Bacillus thuringiensis var. morrisoni PS71M3 ( B.t. PS71M3) and Bacillus thuringiensis var. israelensis PS123D1 ( B.t. PS123D1), have thus far been shown to be active against all mosquitoes tested.

The subject invention also includes asporogenous (spo⁻) mutants of the novel isolates which have substantially the same pesticidal properties as the parent microbes. These mutants are designated Bacillus thuringiensis var. morrisoni PS71M3-69 ( B.t. PS71M3-69) and Bacillus thuringiensis var. israelensis PS123D1-45 ( B.t. PS123D1-45). Advantageously, the novel mutants of the invention are lysis minus, i.e., they do not lyse readily. Procedures for making mutants are disclosed herein.

Further, the invention also includes the treatment of substantially intact B.t. cells of the invention to prolong the pesticidal activity and maintain it in the feeding zone where it is available for ingestion by the target pests when the substantially intact cells are applied to the environment of a target pest. Such treatment can be by chemical or physical means, or a combination of chemical or physical means, so long as the technique does not deleteriously affect the properties of the pesticide, nor diminish the cellular capability in protecting the pesticides. The treated B.t. cell acts as a protective coating for the pesticidal toxin. The toxin becomes available to act as such upon ingestion by a target insect. Since the mutant cells of the subject invention remain intact during extended fermentation periods, the cells can be readily treated by the above procedures. Further, the absence of spores in the mutant microbes provides for a safer insecticide without threatening the environment with spore contamination.

### Detailed Disclosure of the Invention

The novel Bacillus thuringiensis isolates of the subject invention have the following characteristics:

|  | B.t. PS71M3 | B.t. PS123D1 |
|---|---|---|
| Colony morphology | large colony dull surface typical B.t. | same |
| Vegetative cell morphology | typical B.t. | same |
| Flagellar serotype | 8a8b, morrisoni | 14, israelensis |
| Intracellular inclusion | amorphic | amorphic |
| Alkali-soluble proteins (SDS-PAGE) | 144,000 | ≈130,000 |
|  | ≈130,000 | 67,000 |
|  | 67,000 | 27,000 |
|  | 27,000 |  |

The spo⁻ mutants have the same characteristics as listed above for the parent cultures.

Both isolates, and their asporogenous mutants, kill all mosquitoes tested. Bioassays have been done against Aedes aegypti , Aedes dorsalis , Anopheles albomanis , Culex pipens quinquefasciatus and Culex tarsalis .

Bioassay procedure: A dilution of a spore and crystal suspension is added to water in a small cup. Fourth instar larvae are added. Mortality is read after 48 hours.

The mutant cultures disclosed in this application have been deposited in the Agricultural Research Service Patent Culture Collection (NRRL), Northern Regional Research Center, 1815 North University Street, Peoria, Illinois 61604 USA.

| Culture | Repository No. | Deposit Date |
|---|---|---|
| Bacillus thuringiensis var. morrisoni PS71M3-69 | NRRL B-18515 | June 29, 1989 |
| Bacillus thuringiensis var. israelensis PS123D1-45 | NRRL B-18514 | June 29, 1989 |

The B.t. cultures of the subject application can be cultured using standard art media and fermentation techniques. Upon completion of the fermentation cycle, the bacteria can be harvested, if desired, by first separating the B.t. spores and crystals of the parent microbes from the fermentation broth by means well known in the art.

The spo⁻ mutants of the subject application, advantageously, can be cultured and harvested without the added step of separating out spores.

The B.t. cells of the invention can be treated prior to formulation, while substantially intact, to prolong the pesticidal activity and maintain the material in the feeding zone when the cells are applied to the environment of a target pest. Such treatment can be by chemical or physical means, or by a combination of chemical and/or physical means, so long as the technique does not deleteriously affect the properties of the pesticide, nor diminish the cellular capability in protecting the pesticide. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Bouin's fixative and Helly's fixative (See: Humason, Gretchen L., Animal Tissue Techniques, W.H. Freeman and Company, 1967); or a combination of physical (heat) and chemical agents that prolong the activity of the toxin produced in the cell when the cell is applied to the environment of the target pest(s). Examples of physical means are short wavelength radiation such as gamma-radiation and X-radiation, freezing, UV irradiation, lyophilization, and the like. See U.S. Patent No. 4,695,462 for procedures for treating microbial cells. The processes of this patent are incorporated herein by reference thereto.

The B.t. cells of the invention may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about $10^2$ to about $10^4$ cells/mg. These formulations will be administered at about 50 mg (liquid or dry) to 1 kg or more per hectare.

The formulations can be applied to the environment of the dipteran pest(s), e.g., plants, soil or water, by spraying, dusting, sprinkling, or the like.

Following are examples which illustrate procedures, including the best mode, for practicing the invention. These examples should not be construed as limiting. All percentages are by weight and all solvent mixture proportions are by volume unless otherwise noted.

Example 1 - Culturing _B.t.PS71M3_ and _B.t.PS123D1_

A subculture, or starter culture, of B.t. PS71M3 or B.t. PS123D1 can be used to inoculate to following medium known as LB broth:

| Tryptone | 10 gm |
|---|---|
| Yeast extract | 5 gm |
| NaCl | 5 gm |
| 5N NaOH | 0.6 ml |
| Water | 1000.0 ml |

As per standard microbiological techniques, the above medium would be sterilized prior to inoculation and the inoculation would be done using aseptic procedures.

A detailed procedure is as follows:

A series of 150 ml Erlenmeyer flasks containing sterile PWYE medium (peptone 5%, yeast extract 0.1%, NaCl 0.5% in 1 liter of water; adjust pH to 7.5) are inoculated from a petri plate culture of B.t. PS71M3 or B.t. PS123D1. The flasks are incubated at 30°C on a rotary shaker (200 rpm) overnight. From this starter culture, 300 ml of LB broth in a 2-liter flask is inoculated using 7.5 ml of the starter. The LB-broth flasks are incubated under the same conditions as the starter but are harvested after a few hours. The optical density, at 600 nm, is 1.0.

The above procedure can be readily scaled up to large fermentors by procedures well known in the art.

The B.t. cells, obtained in the above fermentation, can be isolated by procedures well known in the art. A frequently-used procedure is to subject the harvested fermentation broth to separation techniques, e.g., centrifugation. Alternatively, the B.t. cells can be treated in the fermentation vessel, without harvesting the cells, as described previously.

Example 2 - Preparation of Asporogenous Lysis Minus Mutants Designated _B.t._ var. _morrisoni_ (PS71M3-69) and _B.t._ var _israelensis_ (PS123D1-45)

The novel asporogenous, lysis minus mutants of the subject invention were prepared as follows:

1. The wild type B.t. var. morrisoni PS71M3 and B.t. israelensis PS123D1 were streaked out onto nutrient agar directly from a frozen (-85°C) ampoule. Ten plates were inoculated.

2. The plates were then incubated at 30°C for 72 hours.

3. The spores were harvested with 7 ml of sterile 0.05 M $PO_4$ buffer, pH 7.0 (7 ml each plate).

4. The suspension was then separated into five 10 ml aliquots.

5. The five aliquots were then exposed to a 70°C water bath for 15 minutes to kill any vegetative cells present.

6. It was to these treated samples that ethylmethane sulfonate (EMS) (obtained from Sigina Chemical Company) was added. Doses were as follows: 0.15 ml; 0.25 ml; 0.35 ml; 0.50 ml.

7. After addition of the EMS, the samples were incubated at 30°C at 350 rpm (rotary shaker) for 18 hours.

8. The treated cultures were then washed twice with 10 ml of sterile distilled water and centrifuged at 8,000 rpm for 5 minutes. Plate counts were done to determine the percent kill.

9. The final pellets were then resuspended in 10 ml of sterile saline and 10 ml of 40% sterile glycerol and stored at $-85°$ C.

10. Lysis minus strains can be identified by screening asporogenous mutants in shake flask medium and selecting those mutants that are still intact and contain toxin crystals at the end of the fermentation.

Ranges:

EMS: 0.15 ml-0.50 ml

% kill: 98-99.999%

EMS exposure time: 15-18 hours

Water bath exposure temp.: 70-80° C

There are other means to obtain asporogenous, lysis minus mutants of Bacillus thuringiensis such as nitrosoguanidine, UV radiation, nitrous acid, site specific mutagenesis, etc.

Example 3 - Preparation of Parent Strains *B.t.* PS71M3 and *B.t.* PS123D1 From Mutant Strains *B.t.* PS71M3-69 and B.t. PS123D1-45, Respectively

The parent strain can be obtained from the mutant under stressed conditions, such as pasteurization. Under the conditions that are described below, the following reversion frequencies have been established:

| PS123D1-45 | $7.92 \times 10^{-11}$ |
|---|---|
| PS71M3D-69 | $1.63 \times 10^{-11}$ |

Statistically, this indicates that one out of $1 \times 10^{11}$ cells will be a revertant. Gel scan analysis of the proteins produced upon outgrowth of the spore-formers (revertants) found after pasteurization was identical to the parents for both strains. The procedure is as follows:

1. Grow mutant culture ( B.t. PS71M3-69 or B.t. PS123D1-45) in 1 liter of dilute medium (L-Broth) for 24 hours on a rotary shaker.

2. Harvest cell suspension and transfer to sterile centrifuge bottles and spin 10 minutes at 8,000 rpm.

3. Remove the supernatant and resuspend the pellets in enough sterile deionized water to bring the volume to 50 ml.

4. Resuspend the pellets and transfer to a sterile flask. Prepare a plate count from this untreated material.

5. Place the flask in an 80° C water bath, shake at 200 rpm for 15 minutes.

6. Remove the flask and plate the suspension on Nutrient agar (50 plates for 50 mls).

7. Incubate the plates for 48 hours at 30° C and microscopically examine any colonies that appear.

8. Subculture any spore-formers onto Nutrient agar and incubate for 72 hours. The reversion frequency is calculated by dividing the numbr of spore-formers by the total number of cells treated.

Example: $(8.9 \times 10^9 \text{ cfu/ml}) \times 50 \text{ ml} = 4.45 \times 10^{11}$ cells treated

3 spore-formers found

$3/4.45 \times 10^{11} = 6.74 \times 10^{-12}$

9. To determine the identity of the spore-formers, grow the "revertants" in liquid fermentation medium parallel to the parent strain.

10. Analyze and compare the proteins produced to those produed by the parent. The "revertants" and the parent strains should have identical banding patterns.

## Claims

1. A microbe selected from Bacillus thuringiensis var. morrisoni PS71M3-69, having the identifying characteristics of deposit NRRL B-18515; Bacillus thuringiensis var. israelensis PS123D1-45, having the identifying characteristics of deposit NRRL B-18514; and the respective parent strains, obtainable under stressed conditions from the preceding strains, and identified as Bacillus thuringiensis var. morrisoni PS71M3 and Bacillus thuringiensis var. israelensis PS123D1.

2. A microbe according to claim 1, which is Bacillus thuringiensis var. morrisoni PS71M3 or PS71M3-69.

3. A microbe according to claim 1, which is Bacillus thuringiensis var. israelensis PS123D1 or PS123D1-45.

4. A stable lysis⁻ spo⁻ cry⁺ Bacillus thuringiensis microbe selected from Bacillus thuringiensis var. morrisoni PS71M3-69, having the identifying characters od deposit NRRL B-18515; and Bacillus thuringiensis var. israelensis PS123D1-45, having the identifying characteristics of deposit NRRL B-18514.

5. A composition of matter comprising a microbe according to any preceding claim, in association with an insecticide carrier.

6. An insecticidal composition comprising substantially intact cells treated in order to have prolonged pesticidal activity and greater persistence in the feeding zone when applied to the environment of a target pest, wherein the insecticide is a polypeptide toxic to dipteran insects, is intracellular, and is produced by a microbe according to any of claims 1 to 4.

7. A method for controlling insect pests, which comprises contacting the pests with a microbe according to any of claims 1 to 4, or with a composition according to claim 5 or claim 6.

8. A method according to claim 7, wherein the insect pests belong to the order Diptera.

9. A method according to claim 7, wherein the insect pests are mosquitos.

10. A method according to claim 9, wherein said mosquitos are selected from Aedes aegypti , Aedes dorsalis , Anopheles albomanis , Culex pipiens quinquefasciatus and Culex tarsalis .

| | DOCUMENTS CONSIDERED TO BE RELEVANT | | EP 90307204.9 |
|---|---|---|---|

| Category | Citation of document with indication, where appropriate, of relevant passages | Relevant to claim | CLASSIFICATION OF THE APPLICATION (Int. Cl.5) |
|---|---|---|---|
| A | EP - A2 - 0 228 228 (MYCOGEN CORP.) * Claims 1,2,9,10 * -- | 1-4,7, 8 | C 12 N 1/20 A 01 N 63/00 //(C 12 N 1/20 C 12 R 1:07) |
| A | EP - A1 - 0 308 199 (MYCOGEN CORP.) * Abstract * -- | 1,7-10 | |
| A | EP - A1 - 0 278 035 (BASF AG) * Claims 1-4,7-10 * ---- | 1,6-9 | |
| | | | **TECHNICAL FIELDS SEARCHED (Int. Cl.5)** |
| | | | C 12 N A 01 N |

The present search report has been drawn up for all claims

| Place of search | Date of completion of the search | Examiner |
|---|---|---|
| VIENNA | 19-10-1990 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)